# EUROPEAN PATENT SPECIFICATION

(11) **EP 3 852 761 B1**
(45) Date of publication and mention of the grant of the patent: **13.05.2026**
(21) Application number: 19780084.0
(22) Date of filing: 18.09.2019
(51) Int. Cl.: A61K 31/513, A61K 31/553, A61K 31/675, A61K 31/683, A61K 45/00, A61K 31/18, A61K 31/535

(54) **AN ADMINISTRATION REGIMEN OF INTEGRASE INHIBITORS FOR PREVENTING HIV INFECTION BY POST-EXPOSURE PROPHYLAXIS**
EIN VERABREICHUNGSSCHEMA FÜR INTEGRASE-INHIBITOREN ZUR VORBEUGUNG EINER HIV-INFEKTION DURCH POSTEXPOSITIONSPROPHYLAXE
UN RÉGIME D'ADMINISTRATION DES INHIBITEURS DE L'INTEGRASE POUR LA PREVENTION DE L'INFECTION PAR LE VIH DANS LE CADRE D'UNE PROPHYLAXIE POST-EXPOSITION

(30) Priority: 19.09.2018 US 201862733536 P
(43) Date of publication of application: 28.07.2021
(73) Proprietor: GILEAD SCIENCES, INC., Foster City, California 94404 (US)
(72) Inventor: BEKERMAN, Elena, Foster City, California 94404 (US); CALLEBAUT, Christian, Foster City, California 94404 (US); MCCALLISTER, Scott, San Francisco, California 94122 (US)
(74) Representative: Carpmaels & Ransford LLP
(86) International application number: PCT/US2019/051681
(87) International publication number: WO 2020/061163

(56) References cited:
- WO-A1-2015/196116
- US-A1- 2015 366 872
- MAYER KENNETH H. ET AL: "Optimal HIV Postexposure Prophylaxis Regimen Completion With Single Tablet Daily Elvitegravir/Cobicistat/Tenofovir Disoproxil Fumarate/Emtricitabine Compared With More Frequent Dosing Regimens", JOURNAL OF ACQUIRED IMMUNE DEFICIENCY SYNDROMES, vol. 75, no. 5, 15 August 2017 (2017-08-15), US, pages 535 - 539, XP093217022, ISSN: 1525-4135, Retrieved from the Internet <URL:https://pmc.ncbi.nlm.nih.gov/articles/PMC5606152/pdf/nihms872895.pdf> DOI: 10.1097/QAI.0000000000001440
- DAVID WOHL ET AL: "Patient-Reported Symptoms Over 48 Weeks Among Participants in Randomized, Double-Blind, Phase III Non-inferiority Trials of Adults with HIV on Co-formulated Bictegravir, Emtricitabine, and Tenofovir Alafenamide versus Co-formulated Abacavir, Dolutegravir, and Lamivudine", 29 June 2018 (2018-06-29), pages 561 - 573, XP055636563, Retrieved from the Internet <URL:https://link.springer.com/content/pdf/10.1007/s40271-018-0322-8.pdf> [retrieved on 20191028]
- ANONYMOUS: "Gilead Announces Phase 3 Results for Investigational Fixed-Dose Combination of Bictegravir, Emtricitabine and Tenofovir Alafenamide for Treatment of HIV", CONFERENCE REPORTS FOR NATAP, IAS 2017: CONFERENCE ON HIV PATHOGENESIS TREATMENT AND PREVENTION, 23 July 2017 (2017-07-23), XP055636249, Retrieved from the Internet <URL:http://www.natap.org/2017/IAS/IAS_04.htm> [retrieved on 20191028]
- ANTHONY MARKHAM: "Bictegravir: First Global Approval.", 21 March 2018 (2018-03-21), XP009516831, Retrieved from the Internet <URL:http://www.ncbi.nlm.nih.gov/pubmed/29564777> [retrieved on 20191028]

## Description

### FIELD

The present disclosure relates to bictegravir, or a pharmaceutically acceptable salt thereof, emtricitabine, or a pharmaceutically acceptable salt thereof, and tenofovir alafenamide, or a pharmaceutically acceptable salt thereof, for use in methods of preventing HIV in a subject.

### BACKGROUND

The HIV/AIDS pandemic has claimed the lives of millions of people, and millions more are currently infected. Antiretroviral therapy has turned HIV infection into a chronic, manageable disease; however, no cure yet exists for HIV. Reduction in the number of new HIV infections is a global goal. To this end, prevention regimens relating to both pre-exposure prophylaxis (PrEP) and post-exposure prophylaxis (PEP) are being explored. Truvada (emtricitabine-tenofovir) is currently the only medication approved for PrEP. Accordingly, new and effective means for preventing HIV infection are needed and the methods described herein are developed to help meet this need.

US2015366872A1 relates to crystalline forms and co-crystals of (2R,5S,13aR)-8-hydroxy-7,9-dioxo-N-(2,4,6-trifluorobenzyl)-2,3,4,5,7,9,13,13a-octahydro-2,5-methanopyrido[1',2':4,5]pyrazino[2,1-b][1,3]oxazepine-10-carboxamide, the pharmaceutical formulations, and the therapeutic uses thereof.

WO2015196116A1 relates to sodium (2R,5S,13aR)-7,9-dioxo-10 -((2,4,6-trifluorobenzyl)carbamoyl)-2,3,4,5,7,9,13,13a-octahydro-2,5-methanopyrido[1',2':4,5] pyrazino[2,1-b][1,3]oxazepin-8-olate Form I.

David Wohl et al. (2018) relates to patient-reported symptoms over 48 weeks among participants in Phase III non-inferiority trials of adults with HIV on co-formulated bictegravir, emtricitabine and tenofovir alafenamide versus co-formulated abacavir, dolutegravir and lamivudine.

Conference report for NATAP, IAS 2017: Conference on HIV Pathogenesis Treatment and Prevention (anonymous) relates to Gilead's announcement of Phase 3 results for the investigational fixed-dose combination of bictegravir, emtricitabine and tenofovir alafenamide for treatment of HIV.

Anthony Markham (2018) relates to the development of bictegravir leading to the first approval of bictegravir/emtricitabine/tenofovir alafenamide as treatment for HIV-1 infection Mayer Kenneth et al. (2017) relates to HIV postexposure prophylaxis regimen completion with single tablet daily elvitegravir/cobicistat/tenofovir disoproxil fumarate/emtricitabine compared with more frequent dosing regimens.

### SUMMARY

The present invention is defined in the appended claims, and it provides bictegravir, or a pharmaceutically acceptable salt thereof, emtricitabine, or a pharmaceutically acceptable salt thereof, and tenofovir alafenamide, or a pharmaceutically acceptable salt thereof, for use in a method of preventing an HIV infection in a subject by post-exposure prophylaxis, the method comprising a first administration within or at 24 hours after exposure of the subject to the HIV, and a second administration within or at 24 hours after the first administration, of the bictegravir, or a pharmaceutically acceptable salt thereof, emtricitabine, or a pharmaceutically acceptable salt thereof, and tenofovir alafenamide, or a pharmaceutically acceptable salt thereof, wherein, in each of the first and second administrations, the bictegravir, or a pharmaceutically acceptable salt thereof, is administered in a dosage of 50 mg or 100 mg, the emtricitabine, or a pharmaceutically acceptable salt thereof, is administered in a dosage of 200 mg and the tenofovir alafenamide, or a pharmaceutically acceptable salt thereof, is administered in a dosage of 25 mg..

### BRIEF DESCRIPTION OF THE DRAWINGS

FIG. 1 shows a representative scheme illustrating the event driven study design of Example 5.
FIG. 2 shows a PrEP/PEP study design schematic further described in Example 6.
FIG. 3 shows survival in the PrEP/PEP study animals as a percent of SHIV-negative animals per group following eight cycles of low-dose rectal challenge (see Example 6).
FIG. 4 shows a PEP study design schematic further described in Example 6.
FIG. 5 shows survival in the PEP study animals as a percent of SHIV-negative animals per group following five rectal challenge (see Example 6).

### DETAILED DESCRIPTION

In some embodiments, the bictegravir is administered as bictegravir sodium.

In some embodiments, the subject may have or be at risk of having the infection. In some embodiments, the subject has been identified as an individual who is at risk of sexual transmission of HIV. In some embodiments, the individual has been identified as a man, transgender man, transgender woman, or woman who has sex with men, a heterosexual men, a heterosexual woman, and or a sex worker. In some embodiments, the individual has been identified as:
- having anal sex with at least two different sexual partners and no consistent condom use over the last 6 months; and/or
- having history of sexually transmitted diseases (STDs) during the last 12 months (*e.g.*, syphilis, gonorrhea, chlamydiae, HBV or HCV infection); and/or
- using psycho-actives drugs during sexual intercourses (*e.g.*, cocaine, gammahydroxybutyric acid (GHB), methylenedioxymethamphetamine (MDMA), mephedrone); and/or
- having sexual intercourse with one or more partners originating from a region with high prevalence of HIV infection (> 1%) *(e.g.,* South America, Sub-Saharan Africa, South-East Asia, Eastern Europe, French Guyana) and no consistent condom use; and/or
- a sex worker; and/or
- having a sexual partner who is an intravenous drug user sharing injection material; and/or
- having an HIV-infected sexual partner with a detectable plasma viral load (*e.g.,* >50 copies (cp)/milliliter (mL)).

In some embodiments, the subject is HIV-negative. In some embodiments, the HIV is HIV-1 and/or HIV-2.

As used herein, the terms "prevention" or "preventing" refers to the administration of a compound, composition, or pharmaceutically salt according to the present disclosure pre- or post-exposure of the human to the virus but before the appearance of symptoms of the disease, and/or prior to the detection of the virus in the blood. The terms also refer to prevention of the appearance of symptoms of the disease and/or to prevent the virus from reaching detectible levels in the blood. The term includes both pre-exposure prophylaxis (PrEP), as well as post-exposure prophylaxis (PEP) and event driven or "on demand" prophylaxis. The term also refers to prevention of perinatal transmission of HIV from mother to baby, by administration to the mother before giving birth and to the child within the first days of life. The term also refers to prevention of transmission of HIV through blood transfusion.

As used herein, "bictegravir" or "BIC" each refer to the integrase inhibitor drug compound (2R,5S,13aR)-8-hydroxy-7,9-dioxo-N-(2,4,6-trifluorobenzyl)-2,3,4,5,7,9,13,13a-octahydro-2,5-methanopyrido[1',2':4,5]pyrazino[2,1-b][1,3]oxazepine-10-carboxamide (IUPAC name) represented by the below structure (Formula (I)). Bictegravir is described in U.S. Patent No.: 9,216,996.

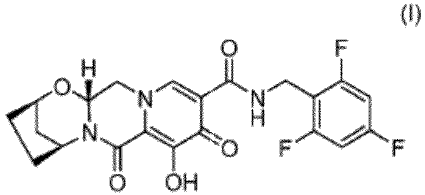

The term bictegravir further includes its pharmaceutically acceptable salts including, for example, its mono sodium salt.

As used herein, "elvitegravir" or "EVG" each refer to the integrase inhibitor drug compound 6-(3-chloro-2-fluorobenzyl)-1-[(2S)-1-hydroxy-3-methylbutan-2-yl]-7-methoxy-4-oxo-1,4-dihydroquinoline-3-carboxylic acid represented by the below structure (Formula (II)). Elvitegravir is described in U.S. Patent No.: 9,216,996.

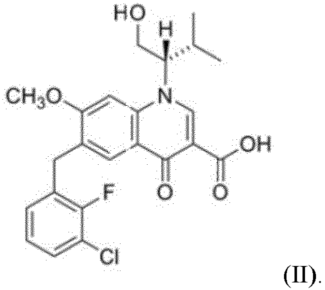

The term elvitegravir further includes its pharmaceutically acceptable salts including, for example, its mono sodium salt.

In the absence of a specific reference to a particular pharmaceutically acceptable salt and/or solvate of the above provided compound of Formula (I) or Formula (II), any dosages, whether expressed in milligrams or as % by weight, should be understood as referring to the amount of the free acid, i.e., the compound of Formula (I) or Formula (II). For example a reference to "50 mg" of bictegravir, or a pharmaceutically acceptable salt thereof, refers to an amount of the compound of Formula (I), or a pharmaceutically acceptable salt thereof, which provides the same amount of the compound of Formula (I) as 50 mg of the compound of Formula (I) free acid. In some embodiments, a dosage referring to 50 mg of bictegravir contains about 52 mg of bictegravir monosodium salt.

As used herein, "tenofovir alafenamide" or "TAF" each refer to the nucleoside analog reverse transcriptase inhibitor drug compound {9-[(R)-2-[[(S)-[[(S)-1-(isopropoxycarbonyl)ethyl] amino]phenoxyphosphinyl]-methoxy]propyl]adenine} having the structure shown below.

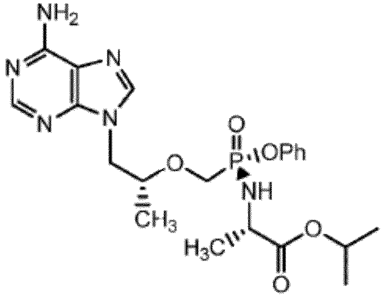

TAF may be associated with fumarate, such as monofumarate and hemifumarate salts or co-crystal (co-formers). See, *e.g.,* U.S. Patent Nos. 7,390,791, 7,803,788, and 8,754,065. It is understood that reference to "TAF" may be inclusive of a co-formers, such as fumarate. TAF is the active pharmaceutical ingredient in Vemlidy^{®} and is a component of the tablets Bictarvy^{®}, Genvoya^{®}, Descovy^{®}, Odefsey^{®}, and Symtuza^{®}.

In the absence of specific reference to a particular pharmaceutically acceptable salt and/or solvate of tenofovir alafenamide, any dosages, whether expressed in milligrams or as a % by weight, should be understood as referring to the amount of tenofovir alafenamide free base. For example reference to 25 mg of tenofovir alafenamide, or a pharmaceutically acceptable salt and/or solvate thereof, refers to an amount of tenofovir alafenamide, or a pharmaceutically acceptable salt and/or solvate thereof, which provides the same amount of tenofovir alafenamide as 25 mg of tenofovir alafenamide free base. In some embodiments, a dosage referring to 25 mg of tenofovir alafenamide contains about 28 mg of tenofovir alafenamide hemifumarate.

As used herein, "tenofovir disoproxil" or "TD" each refer to the compound 9-[(R)-2-[[bis[[(isopropoxycarbonyl)oxy] methoxy]phosphinyl]methoxy]propyl]adenine. TD, a prodrug of tenofovir, may be associated with fumarate, such as monofumarate. See *e.g.,* U.S. Patent Nos. 5,922,695, 5,935,946, and 5,977,089. Tenofovir disoproxil fumarate is referred to as "TDF" and is the active pharmaceutical ingredient in Viread^{®}.

In the absence of specific reference to a particular pharmaceutically acceptable salt and/or solvate of tenofovir disoproxil, any dosages, whether expressed in milligrams or as a % by weight, should be taken as referring to the amount of tenofovir disoproxil free base. For example, reference to 245 mg tenofovir disoproxil, or a pharmaceutically acceptable salt and/or solvate thereof, refers to an amount of tenofovir disoproxil or a pharmaceutically acceptable salt and/or solvate thereof which provides the same amount of tenofovir disoproxil as 245 mg of tenofovir disoproxil free base. In some embodiments, a dosage referring to 245 mg of tenofovir disoproxil contains about 300 mg of tenofovir disoproxil fumarate.

As used herein, "emtricitabine" or "FTC" each refer to the compound 4-amino-5-fluoro-1-[(2R,5S)-2-(hydroxymethyl)-1,3-oxathiolan-5-yl]-1,2-dihydropyrimidin-2-one having the below structure.

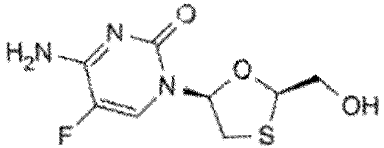

Emtricitabine can be present in dosage forms as a free base or as a pharmaceutically acceptable salt. Additionally, emtricitabine can be present in dosage forms in solvated or unsolvated forms. Typically, emtricitabine is present as a free base.

In the absence of specific reference to a particular pharmaceutically acceptable salt and/or solvate of emtricitabine, any dosages, whether expressed in milligrams or as a % by weight, should be taken as referring to the amount of emtricitabine free base. For example, reference to 200 mg emtricitabine or a pharmaceutically acceptable salt and/or solvate thereof refers to an amount of emtricitabine or a pharmaceutically acceptable salt and/or solvate thereof which provides the same amount of emtricitabine as 200 mg of emtricitabine free base.

As used herein, "cobicistat" or "cobi" each refer to the compound 2,7,10,12-tetraazatridecanoic acid, 12-methyl-13-[2-(1-methylethyl)-4-thiazolyl]-9-[2-(4-morpholinyl)ethyl]-8,11-dioxo-3,6-bis(phenylmethyl)-, 5-thiazolylmethyl ester, (3R,6R,9S)-having the below structure.

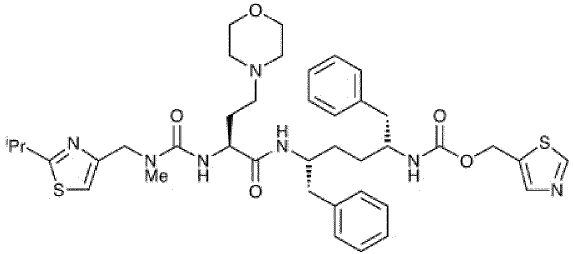

Cobicistat can be present in dosage forms as a free base or as a pharmaceutically acceptable salt. Additionally, cobicistat can be present in dosage forms in solvated or unsolvated forms. Typically, cobicistat is present as a free base. In certain embodiments, cobicistat is present in pharmaceutical compositions in combination with elvitegravir.

In the absence of specific reference to a particular pharmaceutically acceptable salt and/or solvate of cobicistat, any dosages, whether expressed in milligrams or as a % by weight, should be taken as referring to the amount of cobicistat free base. For example, reference to 200 mg cobicistat or a pharmaceutically acceptable salt and/or solvate thereof refers to an amount of cobicistat or a pharmaceutically acceptable salt and/or solvate thereof which provides the same amount of cobicistat as 200 mg of cobicistat free base.

In some embodiments, bictegravir, or a pharmaceutically acceptable salt thereof, is administered in a dosage of about 50 mg/day. In some embodiments, the bictegravir is a pharmaceutically acceptable salt of bictegravir. In some embodiments, the bictegravir is bictegravir sodium salt. In some embodiments, the bictegravir is administered as the sodium salt in a dosage of about 52 mg/day (*e.g.,* 52.5 mg/day). In some embodiments, bictegravir, or a pharmaceutically acceptable salt thereof, is administered in a dosage of about 100 mg/day. In some embodiments, the bictegravir is administered as the sodium salt in a dosage of about 104 mg/day (*e.g.,* 105 mg/day).

In some embodiments, the compound of Formula (I) provided herein, or a pharmaceutically acceptable salt thereof, is administered daily.

As used herein, the terms "event driven" or "event driven administration" refer to administration of the compound of Formula (I), or a pharmaceutically acceptable salt thereof, (1) prior to an event (*e.g.,* 2 hours, 1 day, 2 days, 5 day, or 7 or more days prior to the event) that would expose the individual to HIV (or that would otherwise increase the individual's risk of acquiring HIV); and/or (2) during an event (or more than one recurring event) that would expose the individual to HIV (or that would otherwise increase the individual's risk of acquiring HIV); and/or (3) after an event (or after the final event in a series of recurring events) that would expose the individual to HIV (or that would otherwise increase the individual's risk of acquiring HIV).

The methods disclosed herein involve administration after an event that would expose the individual to HIV or that would otherwise increase the individual's risk of acquiring HIV, namely as post-exposure prophylaxis (PEP). Examples of events that could increase an individual's risk of acquiring HIV include, without limitation, no condom use during anal intercourse with an HIV positive partner or a partner of unknown HIV status; anal intercourse with more than 3 sex partners; exchange of money, gifts, shelter or drugs for anal sex; sex with male partner and diagnosis of sexually transmitted infection; and no consistent use of condoms with sex partner known to be HIV positive. The methods disclosed herein comprise post-exposure prophylaxis (PEP).

The compound of Formula (I), or a pharmaceutically acceptable salt thereof, is administered after exposure of the subject to the HIV.

In some embodiments, the compound of Formula (I), or a pharmaceutically acceptable salt thereof, is administered during the time of HIV-exposure. In some embodiments, the compound of Formula (I) or Formula (II), or a pharmaceutically acceptable salt thereof, is administered daily (*e.g.,* for 7 days) after final exposure to the HIV (*e.g.,* after a period of sexual activity with sex partner known to be HIV positive). In some embodiments, the administration is continued for 1 or 2 days after final exposure to HIV.

In some embodiments, the compound of Formula (I), or a pharmaceutically acceptable salt thereof, is administered as a single dose at 24 hours after exposure of the subject to the HIV. In some embodiments, the compound of Formula (I), or a pharmaceutically acceptable salt thereof, is administered as a single dose at 48 hours after exposure of the subject to the HIV.

Additional examples of PrEP and/or PEP can be found, for example, at the clinical trial summary titled "On Demand Antiretroviral Pre-exposure Prophylaxis for HIV Infection in Men Who Have Sex With Men" (Clinical Trial # NCT01473472); the clinical trial summary titled "Prevention of HIV in Île-de-France" (Clinical Trials # NCT03113123), and at Molina et al, N. Engl. J. Med. 2015, 353:2237-2246.

In some embodiments, , the compound of Formula (I), or a pharmaceutically acceptable salt thereof, is administered 1 to 24 hours, or 1 to 12 hours following an event that would increase the individual's risk of acquiring HIV (*e.g*., following sex or other exposure to the HIV virus). In certain embodiments, the compound of Formula (I), or a pharmaceutically acceptable salt thereof, is administered for 7 days, 14 days, 21 days, 28 days, 30 days, or 45 days following an event that would increase the individual risk of acquiring HIV (*e.g*., following sex or other exposure to the HIV virus). In certain embodiments, the compound of Formula (I) or a pharmaceutically acceptable salt thereof, is administered for 30 days following an event that would increase the individual risk of acquiring HIV (*e.g*., following sex or other exposure to the HIV virus). In certain embodiments, the compound of Formula (I), or a pharmaceutically acceptable salt thereof, is administered less than 1 hour, 2 hours, 3 hours, 4, hours, 5 hours, 6 hours, 7 hours, 8 hours, 9 hours, 12 hours, 18 hours, or 24 hours, following an event that would increase the individual's risk of acquiring HIV (*e.g*., following sex or other exposure to the HIV virus). In certain embodiments, the compound of Formula (I), or a pharmaceutically acceptable salt thereof, is administered for 1 day, 2 days, 3, days 4 days, or 5 days following an event that would increase the individual's risk of acquiring HIV (*e.g*., following sex or other exposure to the HIV virus). In certain embodiments, when the compound of Formula (I), or a pharmaceutically acceptable salt thereof, is administered following an event that would increase the individual's risk of acquiring HIV, it is administered daily following the event.

In certain embodiments, when the compound of Formula (I), or a pharmaceutically acceptable salt thereof, is administered following an event that would increase the individual's risk of acquiring HIV, it is administered twice following the event (*e.g.,* a first administration within a 24 hour period following the event and a second administration at 24 hours following the first administration).

The compound of Formula (I), or a pharmaceutically acceptable salt thereof, is administered after exposure (*e.g.,* after final exposure) of the subject to the HIV (*e.g.,* after a period of sexual activity with sex partner known to be HIV positive).. In some embodiments, the compound of Formula (I), or a pharmaceutically acceptable salt thereof, is administered from 1 hour to 24 hours after exposure (*e.g*., after final exposure) of the subject to the HIV.

In some embodiments, the first administration is performed at 6 hours after exposure of the subject to HIV and the second administration is performed at 30 hours after exposure of the subject to HIV. In some embodiments, the first administration is performed at 12 hours after exposure of the subject to HIV and the second administration is performed at 36 hours after exposure of the subject to HIV. In some embodiments, the first administration is performed at 24 hours after exposure of the subject to HIV and the second administration is performed at 48 hours after exposure of the subject to HIV.

In some embodiments, the method comprises administering to the subject the compound of Formula (I), or a pharmaceutically acceptable salt thereof (*e.g.,* a sodium salt), in a dosage of about 100 mg/day, wherein a first administration is performed within 24 hours after exposure of the subject to the HIV and a second administration is performed within or at 24 hours after the first administration.

In some embodiments, methods for reducing the risk of acquiring HIV (*e.g.,* HIV-1 and/or HIV-2) comprise administration of the compound of Formula (I), or a pharmaceutically acceptable salt thereof, in combination with safer sex practices. In certain embodiments, methods for reducing the risk of acquiring HIV (*e.g.,* HIV-1 and/or HIV-2) comprise administration to an individual at risk of acquiring HIV. Examples of individuals at high risk for acquiring HIV include, without limitation, an individual who is at risk of sexual transmission of HIV.

In some embodiments, the reduction in risk of acquiring HIV is at least about 40%, 50%, 60%, 70%, 80%, 90%, or 95%. In some embodiments, the reduction in risk of acquiring HIV is at least about 75%. In some embodiments, the reduction in risk of acquiring HIV is about 80%, 85%, or 90%.

The present invention as claimed further includes pharmaceutically acceptable salts of the compound of Formula (I). A salt generally refers to a derivative of a disclosed compound wherein the parent compound is modified by converting an existing acid or base moiety to its salt form. A pharmaceutically acceptable salt is one that, within the scope of sound medical judgment, is suitable for use in contact with the tissues of human beings and animals without excessive toxicity, irritation, allergic response, or other problem or complication, commensurate with a reasonable benefit/risk ratio. Examples of pharmaceutically acceptable salts include, but are not limited to, mineral or organic acid salts of basic residues such as amines; alkali or organic salts of acidic residues such as carboxylic acids; and the like. The pharmaceutically acceptable salts of the present disclosure include the conventional non-toxic salts of the parent compound formed, for example, from non-toxic inorganic or organic acids. The pharmaceutically acceptable salts of the present disclosure can be synthesized from the parent compound which contains a basic or acidic moiety by conventional chemical methods. Generally, such salts can be prepared by reacting the free acid or base forms of these compounds with a stoichiometric amount of the appropriate base or acid. Lists of suitable salts are found in Remington's Pharmaceutical Sciences, 17th ed., Mack Publishing Company, Easton, Pa., 1985, p. 1418 and Journal of Pharmaceutical Science, 66, 2 (1977). In some embodiments, the salt is a sodium salt.

The compound of Formula (I), or its salt, can be present in a composition where the composition includes at least one compound other than the compound of Formula (I) or salt of the disclosure.

In some embodiments, the composition comprises bictegravir, or a salt thereof, and one or more additional compounds (*e.g*., one or more additional therapeutic compounds), or salts thereof. Compositions can include mixtures containing the compound of Formula (I), or salt thereof, and one or more solvents, substrates, carriers, etc. In some embodiments, the composition comprises the compound of Formula (I), or salt thereof, in an amount greater than about 25% by weight, for example, greater than about 25% by weight, greater than about 50% by weight, greater than about 75% by weight, greater than about 80% by weight, greater than about 90% by weight, or greater than about 95% by weight.

The present disclosure further includes pharmaceutical compositions comprising the compound of Formula (I), or pharmaceutically acceptable salt thereof, and at least one pharmaceutically acceptable carrier. As used herein, "pharmaceutically acceptable carrier" is meant to refer to any adjuvant, carrier, excipient, glidant, sweetening agent, diluent, preservative, dye/colorant, flavor enhancer, surfactant, wetting agent, dispersing agent, suspending agent, stabilizer, isotonic agent, solvent, or emulsifier which has been approved by the United States Food and Drug Administration as being acceptable for use in humans or domestic animals.

Administration of the compound of Formula (I), or a pharmaceutically acceptable salt thereof, can be carried out via any of the accepted modes of administration of agents for serving similar utilities. The pharmaceutical compositions of the disclosure can be prepared by combining the compound of Formula (I), or a pharmaceutically acceptable salt thereof, with an appropriate pharmaceutically acceptable carrier and, in specific embodiments, are formulated into preparations in solid, semi solid, liquid or gaseous forms, such as tablets, capsules, powders, granules, ointments, solutions, suppositories, injections, inhalants, gels, microspheres, and aerosols. Exemplary routes of administering such pharmaceutical compositions include, without limitation, oral, topical, transdermal, inhalation, parenteral, sublingual, buccal, rectal, vaginal, and intranasal. In a some embodiments, pharmaceutical compositions of the disclosure are tablets. In some embodiments, pharmaceutical compositions of the disclosure are injection (*e.g*., intramuscular (IM) or intraperitoneal (IP)). Pharmaceutical compositions of the disclosure are formulated so as to allow the active ingredients contained therein to be bioavailable upon administration of the composition to a subject. Compositions that will be administered to a subject take the form of one or more dosage units, where for example, a tablet may be a single dosage unit, and a container of the compound of Formula (I), or a pharmaceutically acceptable salt thereof, in aerosol form may hold a plurality of dosage units. Actual methods of preparing such dosage forms are known, or will be apparent, to those skilled in this art; for example, see Remington: The Science and Practice of Pharmacy, 20th Edition (Philadelphia College of Pharmacy and Science, 2000). The composition to be administered will, in any event, contain a therapeutically effective amount of the compound of Formula (I), or a pharmaceutically acceptable salt thereof, for prevention of an HIV infection or reducing the risk of acquiring HIV, as described herein.

In some embodiments, the compound of Formula (I), or a pharmaceutically acceptable salt thereof, is administered orally. In some embodiments, the compound of Formula (I), or a pharmaceutically acceptable salt thereof, is administered parenterally. Parenteral administration includes, but is not limited to, intravenous, intraarterial, subcutaneous, intraperitoneal, intramuscular, intracranial, transdermal, and vaginal administration. Parenteral administration can be administered, for example, in the form of a single bolus dose or by a continuous perfusion pump. In some embodiments, the compound of Formula (I), or a pharmaceutically acceptable salt thereof, is administered to the subject through a medical device. Exemplary medical devices include, but are not limited to, a patch (*e.g.,* a transdermal patch), an implantable device (*e.g.,* an implantable device for metered or sustained release of an active agent; a subdermal device), a syringe, a contraceptive device (*e.g.,* a vaginal ring, an intrauterine device), and the like..

The pharmaceutical compositions disclosed herein can be prepared by methodologies well known in the pharmaceutical art. For example, in certain embodiments, a pharmaceutical composition intended to be administered by injection can prepared by combining the compound of Formula (I), or a pharmaceutically acceptable salt thereof, with sterile, distilled water so as to form a solution. In some embodiments, a surfactant is added to facilitate the formation of a homogeneous solution or suspension. Surfactants are compounds that non-covalently interact with the compound of Formula (I), or a pharmaceutically acceptable salt thereof, so as to facilitate dissolution or homogeneous suspension of the compound in the aqueous delivery system.

The terms "effective amount" or "therapeutically effective amount" refer to an amount of the compound of Formula (I), or other anti-HIV agent, or a pharmaceutically acceptable salt thereof, which when administered to a subject in need thereof, is sufficient to effect preventing an HIV infection or reducing the risk of contracting HIV infection, as described herein. Such an amount would be sufficient to elicit the biological or medical response of a tissue system, or subject that is sought by a researcher or clinician. The amount of the compound of Formula (I) or other anti-HIV agent which constitutes a therapeutically effective amount will vary depending on such factors as the compound, salt, or composition used for administration, the time of administration, the route of administration, the rate of excretion of the compound, the duration of the treatment, the type of disease-state or disorder being treated and its severity, drugs used in combination with or coincidentally with the compound of Formula (I), and the age, body weight, general health, sex and diet of the subject. Such a therapeutically effective amount can be determined routinely by one of ordinary skill in the art having regard to their own knowledge, the state of the art, and this disclosure.

The term "subject" is meant to refer to a human or other mammals such as laboratory animals and household pets (*e.g.,* cats, dogs, swine, cattle, sheep, goats, horses, rabbits), and non-domestic animals such as non-human primates, mammalian wildlife, and the like, that are in need of therapeutic or preventative treatment for a viral infection, such as HIV infection.

### Combination Therapies

Additional therapeutic agents according to the claims are used in combination with the compounds, salts, and compositions described above for preventing an HIV infection in a subject (*e.g*., in a human subject). In some embodiments, the composition comprises the compound of Formula (I), or a pharmaceutically acceptable salt thereof, and the additional therapeutic agents.

The compound of Formula (I), or a pharmaceutically acceptable salt thereof, is administered in combination with an additional therapeutic agent which is emtricitabine, or a pharmaceutically acceptable salt thereof. In some embodiments, the additional therapeutic agent is emtricitabine.

In some embodiments, the emtricitabine, or a pharmaceutically acceptable salt thereof, is administered in a dosage of about 200 mg/day.

The compound of Formula (I), or a pharmaceutically acceptable salt thereof, is also administered in combination with an additional therapeutic agent which is tenofovir alafenamide, or a pharmaceutically acceptable salt thereof. In some embodiments, the tenofovir alafenamide, or a pharmaceutically acceptable salt thereof, is administered in a dosage of about 25 mg/day. In some embodiments, the tenofovir alafenamide is tenofovir alafenamide hemifumarate. In some embodiments, the tenofovir alafenamide hemifumarate is administered in a dosage of about 28 mg/day.

Accordingly, bictegravir, or a pharmaceutically acceptable salt thereof, is administered in combination with a first additional therapeutic agent which is emtricitabine, or a pharmaceutically acceptable salt thereof, and a second additional therapeutic agent which is tenofovir alafenamide, or a pharmaceutically acceptable salt thereof. In some embodiments, the first additional therapeutic agent is emtricitabine and the second additional therapeutic agent is tenofovir alafenamide hemifumarate.

In some embodiments, bictegravir, or a pharmaceutically acceptable salt thereof, is administered in combination with a first additional therapeutic agent which is emtricitabine, or a pharmaceutically acceptable salt thereof, a second additional therapeutic agent which is tenofovir alafenamide, or a pharmaceutically acceptable salt thereof, and a third additional therapeutic agent which is cobicistat, or a pharmaceutically acceptable salt thereof. In some embodiments, the first additional therapeutic agent is emtricitabine, the second additional therapeutic agent is tenofovir alafenamide hemifumarate, and the third additional therapeutic agent is cobicistat.

In certain embodiments, the methods described herein comprise administration of:
(a) bictegravir as a pharmaceutically acceptable salt in a dosage of about 100 mg/day;
(b) emtricitabine in a dosage of about 200 mg/day; and
(c) tenofovir alafenamide as a pharmaceutically acceptable salt in a dosage of about 25 mg/day.

In certain embodiments, the methods described herein comprise administration of:
(a) bictegravir as a pharmaceutically acceptable salt in a dosage of about 50 mg/day;
(b) emtricitabine in a dosage of about 200 mg/day; and
(c) tenofovir alafenamide as a pharmaceutically acceptable salt in a dosage of about 25 mg/day.

In certain embodiments, the methods described herein comprise administration of:
(a) bictegravir sodium in a dosage of about 104 mg/day;
(b) emtricitabine in a dosage of about 200 mg/day; and
(c) tenofovir alafenamide hemifumarate in a dosage of about 28 mg/day.

In certain embodiments, the methods described herein comprise administration of:
(a) bictegravir sodium in a dosage of about 52 mg/day;
(b) emtricitabine in a dosage of about 200 mg/day; and
(c) tenofovir alafenamide hemifumarate in a dosage of about 28 mg/day.

In certain embodiments, the methods described further comprise administration of cobicistat, or a pharmaceutically acceptable salt thereof. In certain embodiments, the methods described further comprise administration of cobicistat, or a pharmaceutically acceptable salt thereof, in a dosage of about 100 mg/day to about 300 mg/day. In certain embodiments, the methods described further comprise administration of cobicistat in a dosage of about 200 mg/day.

In certain embodiments, the reduction in risk of acquiring HIV is at least about 40%, 50%, 60%, 70%, 80%, 90%, or 95%. In certain embodiments, the reduction in risk of acquiring HIV is at least about 75%. In certain embodiments, the reduction in risk of acquiring HIV is about 80%, 85%, or 90%.

In certain embodiments, the components of the composition are administered as a simultaneous or sequential regimen. When administered sequentially, the combination may be administered in two or more administrations.

In certain embodiments, the compound of Formula (I) is combined with the additional therapeutic agents in a unitary dosage form for simultaneous administration to a subject, for example as a solid dosage form for oral administration (*e.g.*, a fixed dose combination tablet).

Co-administration of the compound of Formula (I), or a pharmaceutically acceptable salt thereof, with the additional therapeutic agents generally refers to simultaneous or sequential administration of the compound of Formula (I) and the additional therapeutic agents, such that therapeutically effective amounts of the compound of Formula (I) and the additional therapeutic agents are both present in the body of the subject.

Co-administration includes administration of unit dosages of bictegravir, or a pharmaceutically acceptable salt thereof, before or after administration of unit dosages of the additional therapeutic agents, for example, administration of the compound of Formula (I) or salt thereof within seconds, minutes, or hours of the administration of the additional therapeutic agents. For example, in some embodiments, a unit dose of the compound of Formula (I), or a pharmaceutically acceptable salt thereof, is administered first, followed within seconds or minutes by administration of a unit dose of the additional therapeutic agents. Alternatively, in other embodiments, a unit dose of the additional therapeutic agents is administered first, followed by administration of a unit dose of the compound of Formula (I), or a pharmaceutically acceptable salt thereof, within seconds or minutes. In some embodiments, a unit dose of the compound of Formula (I), or a pharmaceutically acceptable salt thereof, is administered first, followed, after a period of hours (*e.g.,* 1-12 hours), by administration of a unit dose of the additional therapeutic agents. In other embodiments, a unit dose of the additional therapeutic agents is administered first, followed, after a period of hours (*e.g.,* 1-12 hours), by administration of a unit dose of the compound of Formula (I), or a pharmaceutically acceptable salt thereof.

Some aspects of the disclosure will be described in greater detail by way of specific examples. Those of skill in the art will readily recognize a variety of noncritical parameters which can be changed or modified to yield essentially the same results.

### EXAMPLES

### Example 1. Three-arm, two-stage HIV prophylactic vaccine trial with a second randomisation to evaluate the proportion of HIV infections averted by TAF/FTC in comparison to TDF/FTC PrEP

The title example will be performed according to the following protocol:
Design: Phase III, three-arm randomisation comparing each of 2 experimental combination vaccine regimens with placebo control. There will be a second 1:1 randomisation comparing two PrEP regimens, open-label daily TDF/FTC or TAF/FTC. Subjects will receive study PrEP through week 26 after which access to PrEP will revert to local supply. Pre-screening for HIV status will take place as part of a Registration Cohort which will precede and continue in parallel to PrEP vaccination enrollments.
Objectives/aims: 1) Measure HIV incidence during the first 26 weeks (PrEP period); 2) Measure HIV incidence from week 26 to week 74 among subjects that receive all 3 immunizations; 3) Measure adverse events leading to a clinical decision to discontinue PrEP.
Duration: 40 weeks of PrEP per subject (during the immunization period).

The trial can further be modified to replace TAF/FTC with BIC or EVG optionally in combination with FTC and TAF.

### Example 2. Evaluation of pre-exposure prophylaxis (PrEP) initiation, retention and adherence in pregnant and breastfeeding women

The title example will be performed according to the following protocol:
Design: Observational cohort study in 1200 pregnant women who will be recruited at the first antenatal care (ANC) visit (n=600 pregnant women per site).
Objectives/aims: 1) Determine the distribution of women across the PrEP cascade (initiation, retention, and adherence) and outcomes (HIV acquisition, transmission, and adverse events) in a cohort of pregnant and breastfeeding women using quantitative and qualitative approaches; 2) Evaluate subject and provider-level factors associated with the PrEP cascade using quantitative and qualitative approaches; 3) Apply an established mathematical model to simulate the impact of improvement in the PrEP cascade on HIV infections averted (maternal and perinatal).
Sample size: 1200
Duration: 18 months average

### Example 3. Single-arm longitudinal study to offer daily FTC/TDF as PrEP for Periconception Use to HIV-Uninfected Women

The title example is performed according to the following protocol:
Design: Single-arm longitudinal study to offer daily FTC/TDF as PrEP for periconception use to HIV-uninfected women who report plans for pregnancy with an infected or unknown serostatus partner. Women will have the option to take PrEP during pregnancy. PrEP will be offered as part of a safer conception package inclusive of couples-based HIV counselling and testing (CHCT), antiretroviral therapy (ART) for the infected partner, treatment for STIs and safer conception strategies, such as, limiting sex without condoms to peak fertility.
Objectives/aims: 1) Measure uptake of PrEP by women, during periconception and pregnancy follow up; 2) Measure adherence to PrEP using who initiate periconception PrEP and continue PrEP during pregnancy (quarterly plasma concentrations, daily electronic pill cap); 3) Use qualitative methods to explore PrEP adherence promoters and barriers during periconception and pregnancy to inform conceptual framework.
Sample size: 350 HIV-uninfected women; 67 subject enrolled as of August 2018.
Duration: Minimum of 12 months (women not currently pregnant) and a maximum of 24 months (women who become pregnant during the first 12 months).

This study can be modified to replace FTC/TDF with BIC or EVG optionally in combination with FTC and TAF (or TDF).

### Example 4. Immediate or Deferred PrEP for HIV Prevention

The title example is performed according to the following protocol:
Design: Open-label randomized controlled study/
Primary Objective: Compare the frequency and seriousness of adverse events in women and their infants in Arm A against Arm B where Arm A is an allocation to immediate PrEP in pregnancy and Arm B is to have PrEP deferred until after delivery.
Sample size: 842 (1:1); 268 subjects enrolled as of August 2018.

The title trial commenced in October 2017 and is ongoing with anticipated 400 subject visits (repeat study visits) per month.

### Example 5. Evaluation of Genvoya^{®} for Event-Driven PrEP/PEP in Non-Human Primate Model

This study will be performed to establish a minimal effective dosing regimen of Genvoya^{®} (cobicistat/elvitegravir/emtricitabine/tenofovir) for HIV PrEP/PEP "on demand" using a non-human primate (NHP) animal model. PEP regimen is initiated as soon as possible ≤72 hours from exposure. Two study designs are shown below in Table 1.

**Table 1.**

| | | |
|---|---|---|
| **Indication & Usage** | Pre-exposure prophylaxis (PrEP) | PrEP or Post-exposure prophylaxis (PEP) |
| **Target Population** | HIV-negative adults aged 18 years or older males and females | HIV-negative adolescents aged 12 years or older males and females, transgender |
| **Efficacy & Safety** | Same Contraindication/ Warnings and Precautions as GEN label or comparable to oral ARVs | Same Contraindication/ Warnings and Precautions as GEN label or comparable to oral ARVs |
| **Frequency & Dosing** | 1. One single dose 2-24 hrs before having sex and one single dose 24 hrs after | 1. One single dose 2-24 hrs before sex and one single dose 24 hrs after, or 2 single doses within 24 hrs or 48 hrs (2-dose PrEP) |
| | *planned event driven (short course)* | 2. One single dose 2-24 hrs before sex and one single dose 24 hrs after, or 2-7 single doses within 48/72 hrs |
| | | *unplanned event driven (short course)* |
| **Other Attributes** | Specify median # of X sex acts per mo. and/or X partners every two mo. | |

Rhesus macaques of Indian origin are the best characterized and most utilized non-human primate model for HIV transmission (see *e.g.,* Hatziioannou and Evans. Nature Rev Microbiol, 2012). Infection of these animals with SIV recapitulates hallmarks of HIV-1 pathogenesis (Del Prete and Lifson. Curr Top Microbiol Immunol, 2017). SHIV is a chimeric virus bearing R5 tropic HIV-1 envelope, which readily infects macaques and resembles naturally transmitted virus in the human population.

The combination of FTC with TDF or TAF is highly effective at preventing rectal infection with SHIV in rhesus macaques (see *e.g.,* recent published data summarized in Table 2). More recently, this work was extended to the prevention of vaginal viral challenge with SHIV in a pigtail macaque model. Efforts are currently underway to identify short and potent regimens with GEN (E/C/F/TAF) in rhesus macaques. Without being bound by theory, an addition of an INSTI, a drug class which targets a later step in the viral life cycle relative to RT inhibitors, is hypothesized to further improve protection.

**Table 2.**

| | | | | | | | | | | |
|---|---|---|---|---|---|---|---|---|---|---|
| Garcia-Lerma et al. Plos Med, 2008 | Rectal | FTC+TDF (SQ) | | -2h | | +24h | | 14 | 6/6 | 1/18 |
| Garcia-Lerma et al. Sci Trans Med, 2010 | Rectal | FTC+TDF (SQ & PO) | | -2h | | | | 14 | 4/6 | 0/9 |
| | | | -22h | | +2h | | | | 5/6 | |
| | | | | -2h | | +22h | | | 3/6 | |
| | | | | | +2h | +26h | | | 2/4 | |
| Massud, et al. JID, 2016 | Rectal | FTC+TAF (PO) | -24h | | +2h | | | 19 | 6/6 | 0/6 |
| Massud, et al. CROI abstract 2018 | Vaginal | FTC+TAF (PO) | -24h | | +2h | | | 16 | 5/6 | 0/5 |

| | | | | | | | | | | |
|---|---|---|---|---|---|---|---|---|---|---|
| NA = data not available | | | | | | | | | | |

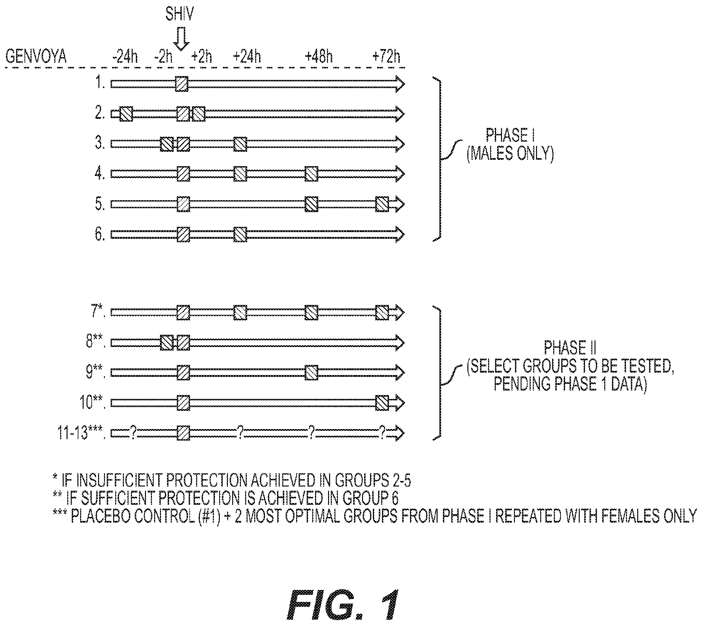

The present study is summarized in Table 3 and Figure 1. Genvoya will be dosed by oral gavage to anesthetized animals as detailed in the study groups schema (FIG. 1). Plasma viral loads will be measured by standard qPCR assay at 2-week intervals to confirm infection. The animals will be monitored for a total of at least 60 days following the last challenge. The resulting rates of protection relative to placebo will determine the minimal effective dosing regimen.

**Table 3.**

| Species | Indian Rhesus Macaques (even split of males and females) |
|---|---|
| N per group | N= 6 (+/-1) |
| Inoculation route | Rectal |
| Virus strain | SHIV 162P3 |
| Total exposures / animal | Single exposure EOW, n=6 total |
| Virus dose | 10-50 TCID₅₀ |
| Route of drug administration | PO. Genvoya pills crushed and dosed in PBS vs placebo. Dose to match human dose. |

The proposed treatment groups are summarized in Figure 1. These may be split into multiple studies and carried out sequentially. This study can be modified to replace Genvoya^{®} with BIC, optionally in combination with FTC and TAF (or TDF).

### Example 6. Evaluation of BIC/FTC/TAF for PEP in Non-Human Primate Model

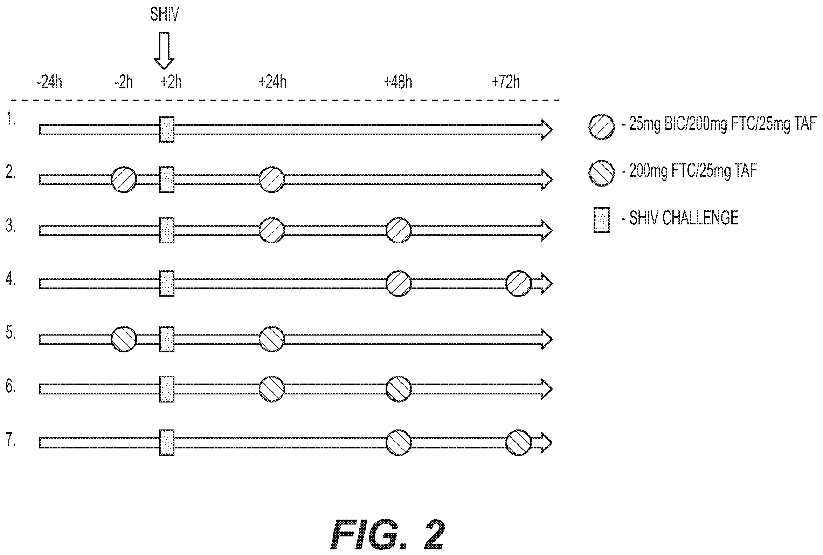

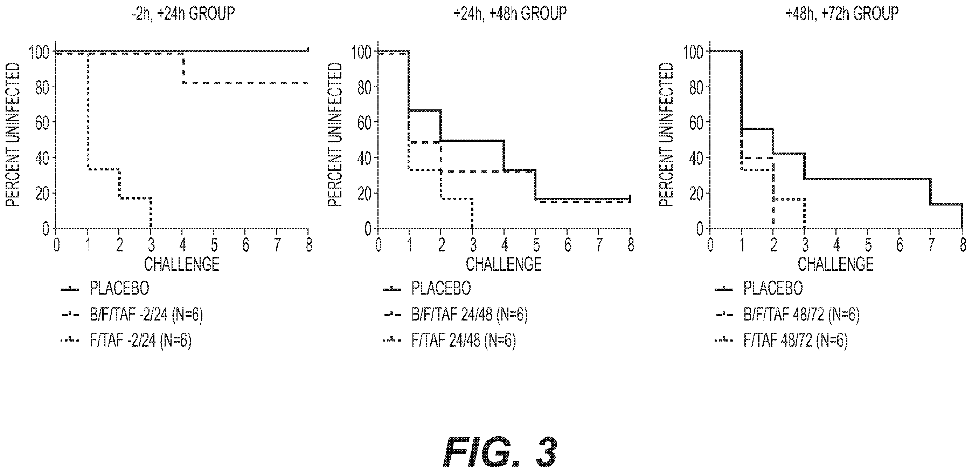

In a previous study, the efficacy of infection prevention was compared with two Antiretroviral Therapy (ART) regimens consisting of either a cocktail of 25 mg BIC (bictegravir), 200 mg FTC (emtricitabine), and 25 mg TAF (tenofovir alafenamide) or 200 mg FTC and 25 mg TAF in a repeat low-dose rectal challenge with SHIV162P3. The animals were stratified into three dosing regimens (groups 2-4 or 5-7), where the two of the above drug combinations were administered either before or after the exposure as summarized in FIG. 2. The rate of infection was established by measuring plasma viremia two weeks post challenge. Following eight challenge cycles, there was near complete protection afforded with -2hr/+24hr regimen (0/6 infected in BIC/FTC/TAF arm and 1/6 in FTC/TAF arm), minimal protection with +24hr/+48hr regimen (5/6 BIC/FTC/TAF arm and 5/6 FTC/TAF arm), and no protection afforded with +48hr/+72hr regimen (6/6 BIC/FTC/TAF arm and 5/5 FTC/TAF arm). Compared to the placebo control group, where all 6 animals became infected within three challenges, significant protection was observed only in the second and fifth groups, where treatment was initiated sooner than 24 hours post-exposure (FIG. 3).

In view of the results described above, a study was performed to determine whether an increase in BIC dose to 100 mg can improve the rate of protection when treatment is initiated 24 hours post-exposure or later. The second objective was to determine whether post-exposure treatment initiation could be protective if initiated earlier than 24 hours post-exposure with either a triple or double-drug regimen (*e.g.* 6 or 12 hours after the exposure).

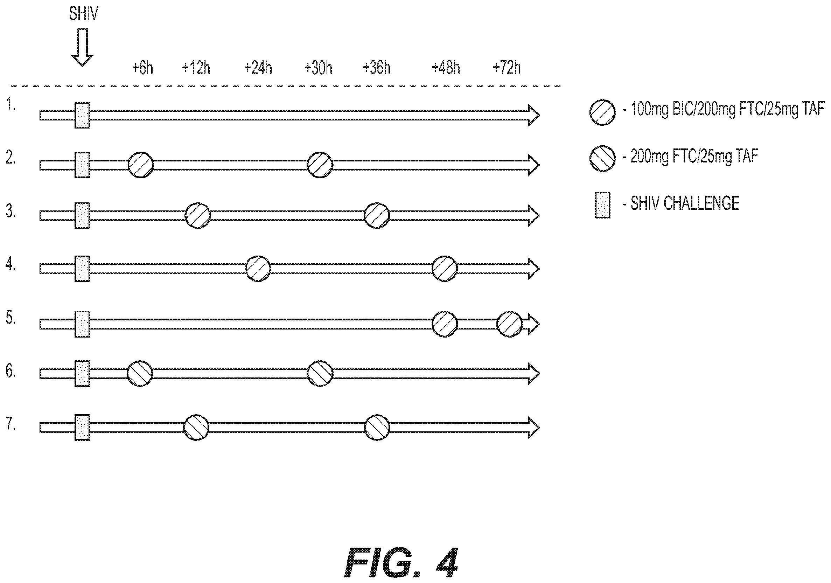

The following dosing regimens were tested (shown schematically in FIG. 4):
1. Placebo control
2. 100mg BIC, 25mg TAF, 200mg FTC dosed at +6, +30 post SHIV challenge
3. 100mg BIC, 25mg TAF, 200mg FTC dosed at +12, +36 post SHIV challenge
4. 100mg BIC, 25mg TAF, 200mg FTC dosed at +24, +48 post SHIV challenge
5. 100mg BIC, 25mg TAF, 200mg FTC dosed at +48, +72 post SHIV challenge
6. 25mg TAF, 200mg FTC dosed at +6, +30 post SHIV challenge
7. 25mg TAF, 200mg FTC dosed at +12, +36 post SHIV challenge

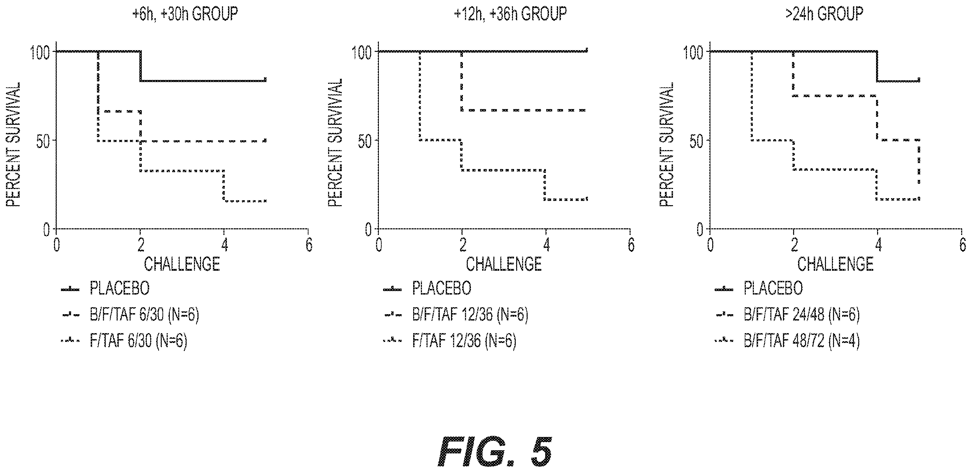

The rate of infection after five challenges (intrarectal route with SHIV162P3) in each of the seven test groups is shown in FIG. 5 and in Table 4, below.

**Table 4. Fraction SHIV+ total**

| | **Group** | **Challenge 1** | **Challenge 2** | **Challenge 3** | **Challenge 4** | **Challenge 5** | **P-value* vs Placebo** |
|---|---|---|---|---|---|---|---|
| **1** | Placebo | 3/6 | 4/6 | 4/6 | 5/6 | 5/6 | |
| **2*** | B/F/TAF 6/30 | 0/6 | 1/6 | 1/6 | 1/6 | 1/6 | 0.0204 |
| **3*** | B/F/TAF 12/36 | 0/6 | 0/6 | 0/6 | 0/6 | 0/6 | 0.0039 |
| **4*** | B/F/TAF 24/48 | 0/6 | 0/6 | 0/6 | 1/6 | 1/6 | 0.0132 |
| **5** | B/F/TAF 48/72 | 0/6 | 1/6 | 1/6 | 2/6 | 3/4 | 0.3314 |
| **6** | F/TAF 6/30 | 2/6 | 3/6 | 3/6 | 3/6 | 3/6 | 0.3009 |
| **7** | F/TAF 12/36 | 0/6 | 2/6 | 2/6 | 2/6 | 2/6 | 0.0608 |

| | | | | | | | |
|---|---|---|---|---|---|---|---|
| * Indicates a claimed regimen | | | | | | | |

Significant protection was observed in groups 2, 3, and 4 (100mg BIC, 25mg TAF, 200mg FTC dosed at +6, +30 post SHIV challenge; +12, +36 post SHIV challenge; and +24, +48 post SHIV challenge).

It should be appreciated that certain features of the disclosure, which are, for clarity, described in the context of separate embodiments, can also be provided in combination in a single embodiment. Conversely, various features of the disclosure which are, for brevity, described in the context of a single embodiment, can also be provided separately or in any suitable subcombination.

The present disclosure provides reference to various embodiments and techniques. However, it should be understood that many variations and modifications may be made while remaining within the scope of the present disclosure.

## Claims

1. Bictegravir, or a pharmaceutically acceptable salt thereof, emtricitabine, or a pharmaceutically acceptable salt thereof, and tenofovir alafenamide, or a pharmaceutically acceptable salt thereof, for use in a method of preventing an HIV infection in a subject by post-exposure prophylaxis, the method comprising a first administration within or at 24 hours after exposure of the subject to the HIV, and a second administration within or at 24 hours after the first administration, of the bictegravir, or a pharmaceutically acceptable salt thereof, emtricitabine, or a pharmaceutically acceptable salt thereof, and tenofovir alafenamide, or a pharmaceutically acceptable salt thereof, wherein, in each of the first and second administrations, the bictegravir, or a pharmaceutically acceptable salt thereof, is administered in a dosage of 50 mg or 100 mg, the emtricitabine, or a pharmaceutically acceptable salt thereof, is administered in a dosage of 200 mg and the tenofovir alafenamide, or a pharmaceutically acceptable salt thereof, is administered in a dosage of 25 mg.

2. The bictegravir, or a pharmaceutically acceptable salt thereof, emtricitabine, or a pharmaceutically acceptable salt thereof, and tenofovir alafenamide, or a pharmaceutically acceptable salt thereof, for use of claim 1, wherein the method comprises:
i) a first administration at 6 hours after exposure of the subject to the HIV; and
ii) a second administration at 30 hours after exposure of the subject to the HIV.

3. The bictegravir, or a pharmaceutically acceptable salt thereof, emtricitabine, or a pharmaceutically acceptable salt thereof, and tenofovir alafenamide, or a pharmaceutically acceptable salt thereof, for use of claim 1, wherein the method comprises:
i) a first administration at 12 hours after exposure of the subject to the HIV; and
ii) a second administration at 36 hours after exposure of the subject to the HIV.

4. The bictegravir, or a pharmaceutically acceptable salt thereof, emtricitabine, or a pharmaceutically acceptable salt thereof, and tenofovir alafenamide, or a pharmaceutically acceptable salt thereof, for use of claim 1, wherein the method comprises:
i) a first administration at 24 hours after exposure of the subject to the HIV; and
ii) a second administration at 48 hours after exposure of the subject to the HIV.

5. The bictegravir, or a pharmaceutically acceptable salt thereof, emtricitabine, or a pharmaceutically acceptable salt thereof, and tenofovir alafenamide, or a pharmaceutically acceptable salt thereof, for use of any preceding claim, wherein the bictegravir, or a pharmaceutically acceptable salt thereof, emtricitabine, or a pharmaceutically acceptable salt thereof, and tenofovir alafenamide, or a pharmaceutically acceptable salt thereof are administered:
(i) simultaneously;
(ii) as a unitary dosage form; and/or
(iii) as a fixed dose combination tablet.

6. The bictegravir, or a pharmaceutically acceptable salt thereof, emtricitabine, or a pharmaceutically acceptable salt thereof, and tenofovir alafenamide, or a pharmaceutically acceptable salt thereof, for use of any one of the preceding claims, wherein the tenofovir alafenamide, or a pharmaceutically acceptable salt thereof is tenofovir alafenamide hemifumarate.

7. The bictegravir, or a pharmaceutically acceptable salt thereof, emtricitabine, or a pharmaceutically acceptable salt thereof, and tenofovir alafenamide, or a pharmaceutically acceptable salt thereof, for use of any one of the preceding claims, wherein the emtricitabine, or a pharmaceutically acceptable salt thereof is emtricitabine.

8. The bictegravir, or a pharmaceutically acceptable salt thereof, emtricitabine, or a pharmaceutically acceptable salt thereof, and tenofovir alafenamide, or a pharmaceutically acceptable salt thereof, for use of any one of the preceding claims, wherein the bictegravir, or a pharmaceutically acceptable salt thereof, emtricitabine, or a pharmaceutically acceptable salt thereof, and tenofovir alafenamide, or a pharmaceutically acceptable salt thereof, are administered orally.

9. The bictegravir, or a pharmaceutically acceptable salt thereof, emtricitabine, or a pharmaceutically acceptable salt thereof, and tenofovir alafenamide, or a pharmaceutically acceptable salt thereof, for use of any one of the preceding claims, wherein the bictegravir, or a pharmaceutically acceptable salt thereof, emtricitabine, or a pharmaceutically acceptable salt thereof, and tenofovir alafenamide, or a pharmaceutically acceptable salt thereof, are administered parenterally, optionally wherein the parenteral administration is selected from sub-cutaneous administration, intramuscular administration, transdermal administration, and vaginal administration.

10. The bictegravir, or a pharmaceutically acceptable salt thereof, emtricitabine, or a pharmaceutically acceptable salt thereof, and tenofovir alafenamide, or a pharmaceutically acceptable salt thereof, for use of any one of the preceding claims, wherein the bictegravir, or a pharmaceutically acceptable salt thereof, is administered to the subject through a medical device, optionally wherein the medical device is selected from a patch, an implantable device, a syringe, and a contraceptive device.

11. The bictegravir, or a pharmaceutically acceptable salt thereof, emtricitabine, or a pharmaceutically acceptable salt thereof, and tenofovir alafenamide, or a pharmaceutically acceptable salt thereof, for use of any one of the preceding claims, wherein the bictegravir is administered as bictegravir sodium.

12. The bictegravir, or a pharmaceutically acceptable salt thereof, emtricitabine, or a pharmaceutically acceptable salt thereof, and tenofovir alafenamide, or a pharmaceutically acceptable salt thereof, for use of any one of the preceding claims, wherein the subject has been identified as an individual who is at risk of sexual transmission of HIV.

13. The bictegravir, or a pharmaceutically acceptable salt thereof, emtricitabine, or a pharmaceutically acceptable salt thereof, and tenofovir alafenamide, or a pharmaceutically acceptable salt thereof, for use of any one of the preceding claims, wherein the HIV is:
(i) HIV-1; or
(ii) HIV-2.

## Patentansprüche

1. Bictegravir oder pharmazeutisch unbedenkliches Salz davon, Emtricitabin oder pharmazeutisch unbedenkliches Salz davon und Tenofoviralafenamid oder pharmazeutisch unbedenkliches Salz davon zur Verwendung bei einem Verfahren zum Vorbeugen einer HIV-Infektion bei einem Individuum durch Post-Exposition-Prophylaxe, wobei das Verfahren eine erste Verabreichung innerhalb von oder 24 Stunden nach Exposition des Individuums gegenüber dem HIV und eine zweite Verabreichung innerhalb von oder 24 Stunden nach der ersten Verabreichung von Bictegravir, oder einem pharmazeutisch unbedenklichen Salz davon, Emtricitabin oder einem pharmazeutisch unbedenklichen Salz davon und Tenofoviralafenamid oder einem pharmazeutisch unbedenklichen Salz davon umfasst, wobei bei jeder der ersten und zweiten Verabreichung das Bictegravir oder ein pharmazeutisch unbedenkliches Salz davon in einer Dosierung von 50 mg oder 100 mg, das Emtricitabin oder ein pharmazeutisch unbedenkliches Salz davon in einer Dosierung von 200 mg und das Tenofoviralafenamid oder ein pharmazeutisch unbedenkliches Salz davon in einer Dosierung von 25 mg verabreicht wird.

2. Bictegravir oder pharmazeutisch unbedenkliches Salz davon, Emtricitabin oder pharmazeutisch unbedenkliches Salz davon und Tenofoviralafenamid oder pharmazeutisch unbedenkliches Salz davon zur Verwendung nach Anspruch 1, wobei das Verfahren Folgendes umfasst:
i) eine erste Verabreichung 6 Stunden nach Exposition des Individuums gegenüber dem HIV; und
ii) eine zweite Verabreichung 30 Stunden nach Exposition des Individuums gegenüber dem HIV.

3. Bictegravir oder pharmazeutisch unbedenkliches Salz davon, Emtricitabin oder pharmazeutisch unbedenkliches Salz davon und Tenofoviralafenamid oder pharmazeutisch unbedenkliches Salz davon zur Verwendung nach Anspruch 1, wobei das Verfahren Folgendes umfasst:
i) eine erste Verabreichung 12 Stunden nach Exposition des Individuums gegenüber dem HIV; und
ii) eine zweite Verabreichung 36 Stunden nach Exposition des Individuums gegenüber dem HIV.

4. Bictegravir oder pharmazeutisch unbedenkliches Salz davon, Emtricitabin oder pharmazeutisch unbedenkliches Salz davon und Tenofoviralafenamid oder pharmazeutisch unbedenkliches Salz davon zur Verwendung nach Anspruch 1, wobei das Verfahren Folgendes umfasst:
i) eine erste Verabreichung 24 Stunden nach Exposition des Individuums gegenüber dem HIV; und
ii) eine zweite Verabreichung 48 Stunden nach Exposition des Individuums gegenüber dem HIV.

5. Bictegravir oder pharmazeutisch unbedenkliches Salz davon, Emtricitabin oder pharmazeutisch unbedenkliches Salz davon und Tenofoviralafenamid oder pharmazeutisch unbedenkliches Salz davon zur Verwendung nach einem vorhergehenden Anspruch, wobei das Bictegravir oder ein pharmazeutisch unbedenkliches Salz davon, Emtricitabin oder ein pharmazeutisch unbedenkliches Salz davon und Tenofoviralafenamid oder ein pharmazeutisch unbedenkliches Salz davon folgendermaßen verabreicht werden:
(i) gleichzeitig;
(ii) als Einheitsdosisform; und/oder
(iii) als Fixdosis-Kombinationstablette.

6. Bictegravir oder pharmazeutisch unbedenkliches Salz davon, Emtricitabin oder pharmazeutisch unbedenkliches Salz davon und Tenofoviralafenamid oder pharmazeutisch unbedenkliches Salz davon zur Verwendung nach einem der vorhergehenden Ansprüche, wobei es sich bei dem Tenofoviralafenamid oder einem pharmazeutisch unbedenklichen Salz davon um Tenofoviralafenamid-Hemifumarat handelt.

7. Bictegravir oder pharmazeutisch unbedenkliches Salz davon, Emtricitabin oder pharmazeutisch unbedenkliches Salz davon und Tenofoviralafenamid oder pharmazeutisch unbedenkliches Salz davon zur Verwendung nach einem der vorhergehenden Ansprüche, wobei es sich bei dem Emtricitabin oder einem pharmazeutisch unbedenklichen Salz davon um Emtricitabin handelt.

8. Bictegravir oder pharmazeutisch unbedenkliches Salz davon, Emtricitabin oder pharmazeutisch unbedenkliches Salz davon und Tenofoviralafenamid oder pharmazeutisch unbedenkliches Salz davon zur Verwendung nach einem der vorhergehenden Ansprüche, wobei das Bictegravir oder ein pharmazeutisch unbedenkliches Salz davon, Emtricitabin oder ein pharmazeutisch unbedenkliches Salz davon und Tenofoviralafenamid oder ein pharmazeutisch unbedenkliches Salz davon oral verabreicht werden.

9. Bictegravir oder pharmazeutisch unbedenkliches Salz davon, Emtricitabin oder pharmazeutisch unbedenkliches Salz davon und Tenofoviralafenamid oder pharmazeutisch unbedenkliches Salz davon zur Verwendung nach einem der vorhergehenden Ansprüche, wobei das Bictegravir oder ein pharmazeutisch unbedenkliches Salz davon, Emtricitabin oder ein pharmazeutisch unbedenkliches Salz davon und Tenofoviralafenamid oder ein pharmazeutisch unbedenkliches Salz davon parenteral verabreicht werden, gegebenenfalls wobei die parenterale Verabreichung aus subkutaner Verabreichung, intramuskulärer Verabreichung, transdermaler Verabreichung und vaginaler Verabreichung ausgewählt ist.

10. Bictegravir oder pharmazeutisch unbedenkliches Salz davon, Emtricitabin oder pharmazeutisch unbedenkliches Salz davon und Tenofoviralafenamid oder pharmazeutisch unbedenkliches Salz davon zur Verwendung nach einem der vorhergehenden Ansprüche, wobei das Bictegravir oder ein pharmazeutisch unbedenkliches Salz davon dem Individuum über eine medizinische Vorrichtung verabreicht wird, gegebenenfalls wobei die medizinische Vorrichtung aus einem Pflaster, einer implantierbaren Vorrichtung, einer Spritze und einer Vorrichtung zur Empfängnisverhütung ausgewählt ist.

11. Bictegravir oder pharmazeutisch unbedenkliches Salz davon, Emtricitabin oder pharmazeutisch unbedenkliches Salz davon und Tenofoviralafenamid oder pharmazeutisch unbedenkliches Salz davon zur Verwendung nach einem der vorhergehenden Ansprüche, wobei das Bictegravir als Bictegravir-Natrium verabreicht wird.

12. Bictegravir oder pharmazeutisch unbedenkliches Salz davon, Emtricitabin oder pharmazeutisch unbedenkliches Salz davon und Tenofoviralafenamid oder pharmazeutisch unbedenkliches Salz davon zur Verwendung nach einem der vorhergehenden Ansprüche, wobei das Individuum als ein Individuum identifiziert wurde, bei dem ein Risiko auf sexuelle Übertragung von HIV besteht.

13. Bictegravir oder pharmazeutisch unbedenkliches Salz davon, Emtricitabin oder pharmazeutisch unbedenkliches Salz davon und Tenofoviralafenamid oder pharmazeutisch unbedenkliches Salz davon zur Verwendung nach einem der vorhergehenden Ansprüche, wobei es sich bei dem HIV um Folgendes handelt:
(i) HIV-1; oder
(ii) HIV-2.

## Revendications

1. Bictégravir, ou un sel pharmaceutiquement acceptable de celui-ci, emtricitabine, ou un sel pharmaceutiquement acceptable de celle-ci, et ténofovir alafénamide, ou un sel pharmaceutiquement acceptable de celui-ci, pour une utilisation dans une méthode de prévention d'une infection par le VIH chez un sujet par prophylaxie post-exposition, la méthode comprenant une première administration au plus 24 heures après l'exposition du sujet au VIH, et une deuxième administration au plus 24 heures après la première administration de bictégravir, ou un sel pharmaceutiquement acceptable de celui-ci, d'emtricitabine ou un sel pharmaceutiquement acceptable de celle-ci, et de ténofovir alafénamide, ou un sel pharmaceutiquement acceptable de celui-ci, dans laquelle, dans chacune des première et deuxième administrations, le bictégravir, ou un sel pharmaceutiquement acceptable de celui-ci, est administré à une dose de 50 mg ou 100 mg, l'emtricitabine, ou un sel pharmaceutiquement acceptable de celle-ci, est administrée à une dose de 200 mg et le ténofovir alafénamide, ou un sel pharmaceutiquement acceptable de celui-ci, est administré à une dose de 25 mg.

2. Bictégravir, ou un sel pharmaceutiquement acceptable de celui-ci, emtricitabine, ou un sel pharmaceutiquement acceptable de celle-ci, et ténofovir alafénamide, ou un sel pharmaceutiquement acceptable de celui-ci, selon la revendication 1, la méthode comprenant :
i) une première administration 6 heures après l'exposition du sujet au VIH ; et
ii) une deuxième administration 30 heures après l'exposition du sujet au VIH.

3. Bictégravir, ou un sel pharmaceutiquement acceptable de celui-ci, emtricitabine, ou un sel pharmaceutiquement acceptable de celle-ci, et ténofovir alafénamide, ou un sel pharmaceutiquement acceptable de celui-ci, selon la revendication 1, la méthode comprenant :
i) une première administration 12 heures après l'exposition du sujet au VIH ; et
ii) une deuxième administration 36 heures après l'exposition du sujet au VIH.

4. Bictégravir, ou un sel pharmaceutiquement acceptable de celui-ci, emtricitabine, ou un sel pharmaceutiquement acceptable de celle-ci, et ténofovir alafénamide, ou un sel pharmaceutiquement acceptable de celui-ci, selon la revendication 1, la méthode comprenant :
i) une première administration 24 heures après l'exposition du sujet au VIH ; et
ii) une deuxième administration 48 heures après l'exposition du sujet au VIH.

5. Bictégravir, ou un sel pharmaceutiquement acceptable de celui-ci, emtricitabine, ou un sel pharmaceutiquement acceptable de celle-ci, et ténofovir alafénamide, ou un sel pharmaceutiquement acceptable de celui-ci, selon l'une quelconque des revendications précédentes, le bictégravir, ou un sel pharmaceutiquement acceptable de celui-ci, l'emtricitabine, ou un sel pharmaceutiquement acceptable de celle-ci et le ténofovir alafénamide, ou un sel pharmaceutiquement acceptable de celui-ci étant administrés :
(i) simultanément ;
(ii) dans une forme posologique unitaire ; et/ou
(iii) sous forme de comprimé en association à dose fixe.

6. Bictégravir, ou un sel pharmaceutiquement acceptable de celui-ci, emtricitabine, ou un sel pharmaceutiquement acceptable de celle-ci, et ténofovir alafénamide, ou un sel pharmaceutiquement acceptable de celui-ci, selon l'une quelconque des revendications précédentes, le ténofovir alafénamide, ou un sel pharmaceutiquement acceptable de celui-ci étant l'hémifumarate de ténofovir alafénamide.

7. Bictégravir, ou un sel pharmaceutiquement acceptable de celui-ci, emtricitabine, ou un sel pharmaceutiquement acceptable de celle-ci, et ténofovir alafénamide, ou un sel pharmaceutiquement acceptable de celui-ci, selon l'une quelconque des revendications précédentes, l'emtricitabine, ou un sel pharmaceutiquement acceptable de celle-ci, étant l'emtricitabine.

8. Bictégravir, ou un sel pharmaceutiquement acceptable de celui-ci, emtricitabine, ou un sel pharmaceutiquement acceptable de celle-ci, et ténofovir alafénamide, ou un sel pharmaceutiquement acceptable de celui-ci, selon l'une quelconque des revendications précédentes, le bictégravir, ou un sel pharmaceutiquement acceptable de celui-ci, l'emtricitabine, ou un sel pharmaceutiquement acceptable de celle-ci, et le ténofovir alafénamide, ou un sel pharmaceutiquement acceptable de celui-ci, étant administrés par voie orale.

9. Bictégravir, ou un sel pharmaceutiquement acceptable de celui-ci, emtricitabine, ou un sel pharmaceutiquement acceptable de celle-ci, et ténofovir alafénamide, ou un sel pharmaceutiquement acceptable de celui-ci, selon l'une quelconque des revendications précédentes, le bictégravir, ou un sel pharmaceutiquement acceptable de celui-ci, l'emtricitabine, ou un sel pharmaceutiquement acceptable de celle-ci, étant administrés par voie parentérale, facultativement, l'administration parentérale étant choisie parmi l'administration sous-cutanée, l'administration intramusculaire, l'administration intramusculaire et l'administration vaginale.

10. Bictégravir, ou un sel pharmaceutiquement acceptable de celui-ci, emtricitabine, ou un sel pharmaceutiquement acceptable de celle-ci, et ténofovir alafénamide, ou un sel pharmaceutiquement acceptable de celui-ci, selon l'une quelconque des revendications précédentes, le bictégravir, ou un sel pharmaceutiquement acceptable de celui-ci, étant administré au sujet par l'intermédiaire d'un dispositif médical, facultativement, le dispositif médical étant choisi parmi un patch, un dispositif implantable, une seringue et un dispositif contraceptif.

11. Bictégravir, ou un sel pharmaceutiquement acceptable de celui-ci, emtricitabine, ou un sel pharmaceutiquement acceptable de celle-ci, et ténofovir alafénamide, ou un sel pharmaceutiquement acceptable de celui-ci, selon l'une quelconque des revendications précédentes, le bictégravir étant administré sous forme de bictégravir sodique.

12. Bictégravir, ou un sel pharmaceutiquement acceptable de celui-ci, emtricitabine, ou un sel pharmaceutiquement acceptable de celle-ci, et ténofovir alafénamide, ou un sel pharmaceutiquement acceptable de celui-ci, selon l'une quelconque des revendications précédentes, le sujet ayant été identifié comme un individu présentant un risque de transmission sexuelle du VIH.

13. Bictégravir, ou un sel pharmaceutiquement acceptable de celui-ci, emtricitabine, ou un sel pharmaceutiquement acceptable de celle-ci, et ténofovir alafénamide, ou un sel pharmaceutiquement acceptable de celui-ci, selon l'une quelconque des revendications précédentes, le VIH étant :
(i) VIH-1 ; ou
(ii) VIH-2.
